# EUROPEAN PATENT APPLICATION

(11) **EP 1 726 648 A1**
(43) Date of publication of application: **29.11.2006**
(21) Application number: 05721539.4
(22) Date of filing: 18.03.2005
(51) Int. Cl.: C12N 15/09, C12Q 1/68, G01N 37/00

(54) **DNA ARRAY AND METHOD OF DETECTING SINGLE NUCLEOTIDE POLYMORPHISM**

(30) Priority: 19.03.2004 JP 2004081034
(71) Applicant: TOYOBO CO., LTD., Osaka-shi Osaka 530-8230 (JP); Ngk Insulator, Ltd., Nagoya-shi, Aichi 467-8530 (JP)
(72) Inventor: KAWASE, Mitsuo c/o NGK Insulators, Ltd., Nagoya-shi, Aichi 467-8530 (JP); YOSHIDA, Yasuko c/o NGK Insulators, Ltd., Nagoya-shi, Aichi 467-8530 (JP); YAMADA, Kazunari c/o NGK Insulators, Ltd., Nagoya-shi, Aichi 467-8530 (JP); TAKARADA, Yutaka c/o Toyobo Co., Ltd. Tsuruga, Tsuruga-shi, Fukui 914-0047 (JP); HASHIMOTO, Kouzo c/o Toyobo Co., Ltd., Osaka-shi, Osaka 530-8230 (JP)
(74) Representative: Bufton, Karen A.J.
(86) International application number: PCT/JP2005/005612
(87) International publication number: WO 2005/090565

(57) **Abstract**

A DNA array for detecting a single nucleotide polymorphism of a gene, which comprises, on a solid support, a first probe spots group consisting of one or more probe spots each containing one or more probes hybridizable with a polynucleotide of the gene, in which the probes are exactly complementary to the first polymorphism pattern of the gene, and a second probe spots group consisting of one or more probe spots each containing one or more probes hybridizable with the polynucleotide of the gene, in which the probes are exactly complementary to the second polymorphism pattern of the gene, wherein probe lengths in the probe spots is different from each other. This DNA array enables more exact SNP detection.

## Description

### Technical Field

The invention of this application relates to a DNA array and a method for detecting a single nucleotide polymorphism. More specifically, the invention of this application relates to a DNA detectable a single nucleotide polymorphism more exactly, and a method for detecting a single nucleotide polymorphism using this DNA array.

### Background Art

On the "post genome", a new technology for detecting nucleotide species in nucleotide sequences exactly, efficiently and at low cost has been in demand. For example, SNP (Single Nucleotide Polymorphism) is a polymorphism of the highest frequency that exists in human genome at a ratio of approximately 0.1% (approximately 1 nucleotide of 1,000 nucleotides). That is, this single nucleotide polymorphism means a state in which a single nucleotide of a genomic gene is replaced with another nucleotide, whereby, for example, a G-C nucleotide pair is provided in wild type, whereas an A-T nucleotide pair is provided in polymorphism pattern. Further, there are cases where both alleles of a diploid chromosome are polymorphism pattern (homozygotic polymorphism) and where one allele is a wild type and another is a polymorphism pattern (heterozygotic polymorphism).

Mutation of a single nucleotide might cause, for example, synthesis of a mutant amino acid by codon mutation (missense mutation) or synthesis of an incomplete protein by formation of a termination codon (nonsense mutation). Accordingly, it is being clarified that the presence or absence of SNP is associated with various diseases (for example, SNP of p53 gene associated with lung cancer: non-Patent Document 1), and the significance of exactly judging the presence or absence of SNP (SNP typing) for diagnosis, gene therapy or the like has been strongly recognized. Moreover, SNP is a useful polymorphism marker in search of a gene related with susceptibility to disease or drug reactivity, and it has attracted much interest as an important gene information for tailor-made medical care.

As a SNP typing method, "a method using hybridization efficiency", "a method using enzyme recognition efficiency", "a method using an electrical procedure" and the like have been known. Especially, the method using hybridization efficiency has variously applied to DNA arrays (refer to, for example, Patent Documents 1 to 4 and non-Patent Documents 2 and 3). For example, in non-Patent Document 4, an example of detecting BRCA1 gene SNP with a DNA array has been reported.

In this DNA array for SNP detection, for example, a first probe spot complementary to a wild type sequence of a target gene and a second probe spot complementary to a single nucleotide polymorphism sequence of the gene are located on a solid support. In SNP detection, a target gene cDNA prepared by a method such as PCR amplification using a fluorescence-labeled primer is contacted with the DNA array. When the target gene is a wild type, the labeled cDNA is hybridized with the first probe, and a fluorescent signal is obtained from only the first probe spot (homozygotic wild type pattern). Meanwhile, when both alleles of the target gene are SNPs, a fluorescent signal is obtained from only the second probe spot (homozygotic SNP pattern). When one allele thereof is SNP, fluorescent signals of the same extent are obtained in both of the first probe spot and the second probe spot (heterozygotic SNP pattern).
Patent Document 1: Specification of U.S. Patent No. 5,474,796
Patent Document 2: Specification of U.S. Patent No. 5,605,662
Patent Document 3: Pamphlet of International Publication No. 95/251116
Patent Document 4: Pamphlet of International Publication No. 95/35505
non-Patent Document 1: Biros et al. Neoplasma 48(5): 407-11, 2001
non-Patent Document 2: Schena, M. et al., Proc. Natl. Acad. Sci. USA. 93:10614-10619, 1996
non-Patent Document 3: Heller, R. A. et al., Proc. Natl. Acad. Sci. USA 94:2150-2155, 1997
non-Patent Document 4: Hacia JG et al. Nat. Genet. 14:441-447, 1996

### Disclosure of the Invention

In SNP detection using the DNA array, it is not easy to clearly differentiate a wild type pattern, a homozygotic SNP pattern and a heterozygotic SNP pattern using a fluorescent signal of each probe spot. That is, there is a difference by only one nucleotide between labeled cDNAs derived from a wild type gene and a polymorphism gene. Accordingly, many of wild type cDNAs are hybridized with a wild type probe (first probe) completely complementary thereto. However, some of them are hybridized also with a second probe different in one nucleotide. Likewise, some of polymorphism cDNAs are hybridized also with the first probe. The SNP detection with the DNA array is conducted by comparing fluorescent signals of the first probe spot and the second probe spot. However, the comparison in signal intensity therebetween is sometimes difficult. Consequently, erroneous SNP judgement has been conducted disadvantageously.

Under these circumstances, the invention of this application aims to provide a DNA array capable of more exact SNP detection and a method for detecting SNP using this DNA array.

Further, the invention of this application aims to provide a method for detecting a probe length capable of more exact detection in the SNP detection using the DNA array, and a DNA array having a probe with a specific length determined by this method.

The inventors of this application have found that subject genes intended for SNP detection are different from each other in probe length capable of obtaining an optimum marker signal for exactly judging an SNP pattern. The invention has been completed by this finding.

This application provides, as a first invention for solving the foregoing problems, a DNA array for detecting a single nucleotide polymorphism of a gene, which comprises, on a solid support,
a first probe spots group consisting of one or more probe spots each containing one or more probes hybridizable with a polynucleotide of the gene, in which the probes are exactly complementary to the first polymorphism pattern of the gene, and
a second probe spots group consisting of one or more probe spots each containing one or more probes hybridizable with the polynucleotide of the gene, in which the probes are exactly complementary to the second polymorphism pattern of the gene,
wherein probe lengths in the probe spots is different from each other.

In the DNA array of the first invention, a preferable embodiment is that the first probe spot group and the second probe spot group each comprise from 2 to 10 probe spots.

In the foregoing embodiment of the first invention, a preferable embodiment is that from 2 to 10 probe spots are arranged in the order of the probe length.

This application provides, as a second invention, a kit for detecting a single nucleotide polymorphism of a gene, the kit comprising at least the DNA array of the first invention.

This application provides, as a third invention, a method for detecting a single nucleotide polymorphism of a gene, which uses the DNA array of the first invention, and comprises the following steps of,
(a) preparing a labeled polynucleotide from the gene;
(b) contacting the labeled polynucleotide with the DNA array; and
(c) measuring signals from the labeled polynucleotide hybridized with the probes on the DNA array.

In the method of the third invention, a preferable embodiment is that in the step (c) the respective signals of the first probe spots group and the second probe spots group are compared.

This application provides, as a fourth invention, a method for determining an appropriate probe length for detecting a single nucleotide polymorphism of a gene, which uses the DNA array of the first invention, comprises the following steps of,
(a) preparing a labeled polynucleotide from the gene;
(b) contacting the labeled polynucleotide with the DNA, array; and
(c) measuring signals from the labeled polynucleotide hybridized with each probe on the DNA array, and
   determining a probe length satisfying the following criteria as an appropriate probe length for detecting the single nucleotide polymorphism of the gene:
   (i) the signal is observed in the first probe spot group in the case of the gene being homozygotic first polymorphism pattern,
   (ii) the signal is observed in the second probe spot group in the case of the gene being homozygotic second polymorphism pattern, and
   (iii) the signals of the same extent are observed in both of the first probe spot group and the second probe spot group in the case of the gene being heterozygotic polymorphism pattern.

This invention provides, as a fifth invention, a DNA array for detecting a single nucleotide polymorphism of a gene, which comprises, on a solid support,
a first probe spot containing one or more probes exactly complementary to the first polymorphism pattern of the gene, and
a second probe spot containing one or more probes exactly complementary to the second polymorphism pattern of the gene,
wherein the probe length is one determined by the method of the fourth invention.

The DNA array of the first invention has the probe spots with different probe lengths which are more exactly hybridizable with different single nucleotide polymorphism patterns. Accordingly, the more exact SNP detection is enabled by the method of the third invention using the DNA array of the first invention.

An appropriate probe length is determined for each gene intended for SNP detection by the method of the fourth invention. This method provides the DNA array of the fifth invention containing the probe spots with the probe length appropriate for the SNP detection of gene.

In the invention of this application, the "single nucleotide polymorphism" refers to, for example, a single nucleotide mutation that is different from a registered sequence of a gene of database. Accordingly, a gene's sequence registered in database does not necessarily mean a wild type (normal type), nor is a gene with single nucleotide mutation not necessarily a mutant gene. However, regarding a gene in which single nucleotide mutation is known to be related with diseases, a wild type may be defined as "a normal type" and a single nucleotide polymorphism as "a mutant type". In the invention of this application, the "first polymorphism pattern" and the "second polymorphism pattern" basically do not mean the "wild type" and the "mutation type". In the following description, the first polymorphism pattern is defined as the pattern of a gene's sequence registered in database, and the second polymorphism pattern as the pattern of a sequence in which a single nucleotide in the sequence of the first polymorphism pattern is substituted with another nucleotide.

In the invention of this application, the "polynucleotide of gene" specifically means a genomic DNA, an mRNA transcribed from the genomic DNA or a cDNA synthesized from the mRNA of a gene for SNP detection. This polynucleotide is a molecule with bonded plural nucleotides, preferably 30 or more nucleotides, more preferably 50 or more nucleotides.

A specific construction of each of the inventions of this application is described in detail in the description of embodiments of the invention or Examples. The terms and concepts according to the invention are within those ordinarily used in the technical field concerned except those specifically defined. Further, various techniques which are used in practicing the invention can be performed by those skilled in the art easily and surely on the basis of known documents or the like except techniques whose original sources are indicated in particular. For example, genetic engineering and molecular biological techniques are described in Sambrook and Maniatis, in Molecular Cloning-A Laboratory Manual, Cold Spring Harbor Laboratory Press, New York, 1989; Ausubel. F. M. et al., Current Protocols in Molecular Biology, John Wiley & Sons, New York, N.Y., 1995 and the like.

### Brief Description of the Drawings

Fig. 1 is a schematic view showing an example of the structure of DNA array of the invention.
Fig. 2 is a schematic view showing another example of the structure of DNA array of the invention.
Fig. 3 is a fluorescent image showing an example of detecting SNP of gene GNB3 with the DNA array of the invention.
Fig. 4 is a fluorescent image showing an example of detecting SNP of gene MTHFR with the DNA array of the invention.
Fig. 5 is a fluorescent image showing an example of detecting SNPs of gene CYP 2C19-2 and gene CYP 2C19-3 with the DNA array of the invention.

### Best Mode for Carrying Out the Invention

In a DNA array of the first invention, a first probe spots group comprising one or more probes and a second probe spots group comprising one or more probes are provided on a solid support. Each of the probe constituting the first probe spots group is hybridizable with a polynucleotide of gene to be analyzed and is exactly complementary to the first polymorphism pattern of the gene. Each of the probe constituting the second probe spots group is hybridizable with the polynucleotide of gene and is exactly complementary to the second polymorphism pattern of the gene. The probe spots constituting the first probe spots group and the second probe spots group comprise probes different in length respectively.

In more detail, the "probe spot" refers to a region in which a group of one or more probes, preferably a group of from 10³ to 10¹³ probes of the same type (probes having the same sequence and the same length) exists separately from other probe spots. The "probe spots group" means a group of probe spots comprising probes having the same sequence and different in length. In one preferable embodiment, this group comprises from 2 to 10 probe spots. Further, a preferable embodiment is that these probe spots are arranged in line in the order of length of the probes constituting the same. The probe spots group arranged in line in this manner is sometimes referred to as a "probe spots row". In the first probe spots row and the second probe spots row, the probe spots having the same probe length may be opposite to each other.

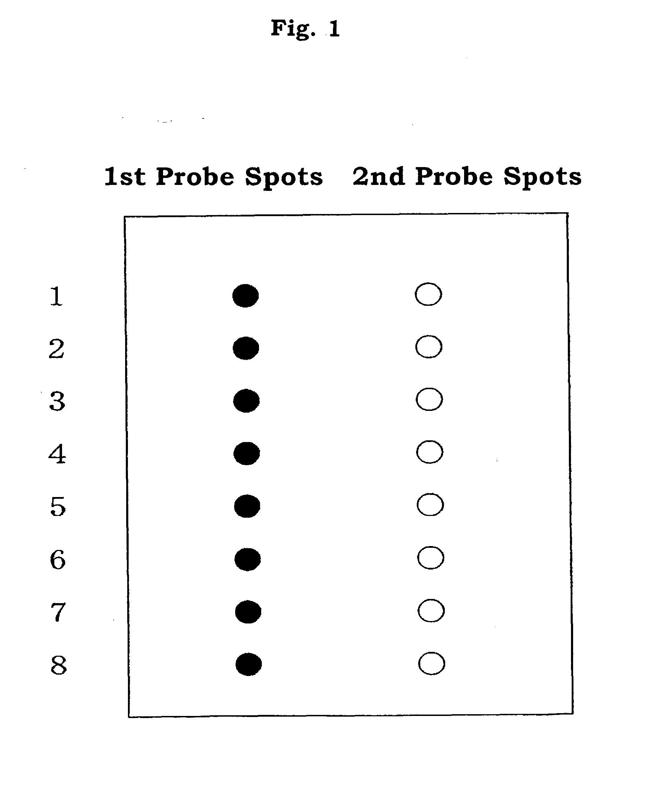

Fig. 1 is an example of the structure of the DNA array of the first invention. In Fig. 1, the row of the first probe spots (black circles) and the row of the second probe spots (white circles) each comprise 8 probe spots. In the respective probe spots, the probes contained therein are arranged in line in the first to eighth steps in the long→short order. That is, in the the first probe spots row and the second probe spots row, the probes in the first step are the n-number of nucleotides (n-mer). From the probes of the second steps, n-1 mer, n-2 mer, n-3 mer, n-4 mer, n-5 mer, n-6 mer and n-7 mer are provided in order. In this case, "n" is from 10 to 100, preferably from 20 to 50.

The "order of length" may be a long→short order or a short→long order. The difference in probe length may be a difference by one nucleotide or a difference by two or three nucleotides. In the example of Fig. 1, the respective spots of the first probe spots row and the second probe spots row are arranged longitudinally (from top to bottom), while they may be arranged laterally (from right to left).

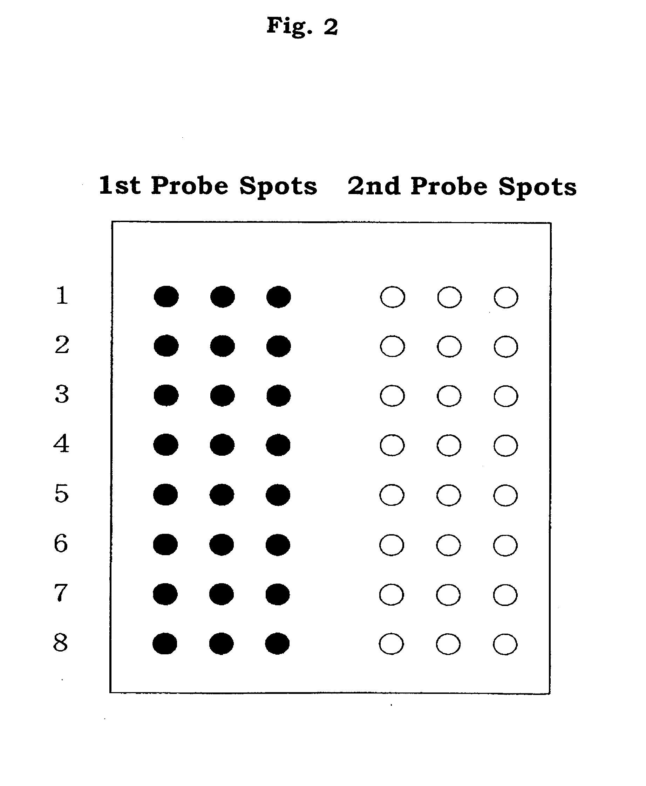

Further, from 2 to 5 probe spots comprising the same probes (probes with the same sequence and the same length) may be arranged in line. For example, in Fig. 2, in each step of the first probe spots row and the second probe spots raw, 3 probe spots comprising the same probes are arranged in parallel. That is, the presence of plural probe spots comprising the same probes can exclude an influence on the number of probes contained in the individual probe spots by slight change.

The DNA array of the first invention can be prepared similarly to a usual DNA array except that the foregoing probes spots groups (preferably probe spots rows) are arranged. As a method for producing a DNA array, a method in which probes are directly synthesized on a surface of a solid support (on-chip method) and a method in which probes previously prepared are fixed on a surface of a solid support have been known. It is advisable that the DNA array of the invention is prepared by the latter method. When probes previously prepared are fixed on a surface of a solid support, probes having a functional group introduced therein is synthesized, and the probes are spotted on a surface-treated solid support and covalently bonded thereon (for example, Lamture, J.B. et al. Nucl. Acids Res. 22:2121-2125, 1994; Guo, Z. et al. Nucl. Acids Res. 22:5456-5465, 1994). The probes are generally covalently bonded on a surface-treated solid support via a spacer or a crosslinker. A method in which small pieces of polyacrylamide gel are aligned on a glass surface and probes are covalently bonded thereon (Yershow, G. et al. Proc. Natl. Acad. Sci. USA 94:4913, 1996) or a method in which probes are bonded on a solid support coated with poly-L-lysine (JP-A-2001-186880) is also known. Further, a method is known in which a microelectrode array is formed on a silica microarray, an agarose impregnation layer containing streptoadipin is formed on the electrode to provide a reaction site, this site is positively charged to fix biotinized probes and the charge of the site is controlled to allow strict hybridization at high speed (Sosnowski, R. G. et al. Proc. Natl. Acad. Sci. USA 94:1119-1123, 1997). The DNA array of the invention can be produced by any of these methods. When spotting is conducted by dropping probes on a surface of a solid support, a pin method can be employed (for example, U. S. Patent No. 5,807,5223). However, it is advisable to employ an ink jet method disclosed in JP-A-2001-116750 or JP-A-2001-186881 for forming spots of a uniform and fixed shape. In this ink jet method, the number of probes contained in individual probe spots can be equalized, so that a difference in hybridization due to a difference in probe length can exactly be measured. It is further recommendable for desirable spot formation that multi-spotting is conducted as disclosed in JP-A-2001-186880 or a probe solution (solution containing a moisturizing substance) having a composition disclosed in WO 03/038089 A1 is used.

After the spotting, cooling, addition of water to spots (kept at humidity of up to 80% for a prescribed period of time), fixing treatment by drying via burning may be conducted to fix spots on a solid support, whereby a DNA array can be completed.

As the solid support of the DNA array, a glass (slide glass) which is used in an ordinary DNA array as well as plastics, silicone, ceramics and the like are available.

The second invention is a kit for detecting a single nucleotide polymorphism, the kit comprising the DNA array. The kit can be formed with the DNA array, PCR primers, a PCR buffer, dNTP, MgCl₂, Taq DNA polymerase and the like.

The method of the third invention is a method for detecting a single nucleotide polymorphism of a gene, which uses the DNA array of the first invention, and indispensably comprises the following steps of,
(a) preparing a labeled polynucleotide from the gene;
(b) contacting the labeled polynucleotide with the DNA array; and
(c) measuring signals from the labeled polynucleotide hybridized with the probes on the DNA array.

The gene in step (a) is one in which the presence of SNP is known, and its labeled polynucleotides can be prepared as a PCR product (cDNA) from a genomic gene or a total RNA isolated from a subject using a primer set disclosed in, for example, a SNP database (for example, http://SNP.ims.u-tokyo.ac.jp/index,ja.html). In the PCR amplification, a labeling primer (for example, a primer with cyanine organic pigment such as Cy3 or Cy5) is incorporated to form the labeled polynucleotides.

In step (b), the labeled polynucleotides are contacted with the DNA array, and are hybridized with the probes of the DNA array. The hybridization can be conducted by spotting on the DNA array the labeled polynucleotides aqueous solution poured on a 96-well or 384-well plastic plate. A spotting amount can be from 1 to 100 nl. It is advisable to conduct the hybridization at from room temperature to 70°C for from 1 to 20 hours. After completion of the hybridization, the DNA array is washed with a mixed solution of a surfactant and a buffer to remove an unreacted labeled polynucleotides. As a surfactant, sodium dodecylsulfate (SDS) is preferably used. As a buffer, a citrate buffer, a phosphate buffer, a borate buffer, a Tris buffer, a Good's buffer or the like can be used. A citrate buffer is preferably used.

In step (c), signals from the labeled polynucleotides hybridized with the probes are measured. From the signals, SNP is detected in the following manner, for example.

First, a cut-off value of the resulting signal is as 20,000. A signal ratio of the first probe spots group and the second probe spots group (first/second ratio) is calculated, from the signals at the spots where signals of at least one of the first probe spot group and the second probe spots group is 20,000 or more, and then polymorphism is judged as follows.
(1) When the signal ratio is >5, the tested gene is judged to be the homozygotic first polymorphism pattern.
(2) When the signal ratio is <0.2, the tested gene is judged to be the homozygotic second polymorphism pattern.
(3) When the signal ratio is at least 0.2 and at most 5, the tested gene is judged to be a heterozygotic polymorphism pattern.

For another judgement protocol, for example, using the DNA array of Fig. 1, the case where signals are observed in all of 8 spots of the first probe spots row and in 4 spots of the second probe spots row corresponds to the above (1), i.e., the tested gene is judged to be a homozygotic first polymorphism pattern. Alternatively, the case where the same number of signaling spots is observed from the first probe spots row and the second probe spots row but the sum of signal intensities in the first probe spots row is larger than that in the second probe spots row also corresponds to the above (1), i.e, the tested gene is judged to be a homozygotic first polymorphism pattern.

The judgement criterion (3) above may be that the number of signaling spots is the same and the sum of signal intensities thereof is 30% or less, preferably 20% or less, more preferably 10% or less.

That is, in conventional methods, values of signal intensities of a single first probes spot and a single second probes spot are compared, whereas in the method of the third invention, the numbers of spots giving much more signals are measured from probe spots groups each comprising plural spots, and are compared. Consequently, this method can detect SNP with far higher precision than in the conventional methods.

The preparation of the labeled polynucleotides and the hybridization procedure in the third invention are described in many patent documents and non-patent documents including, for example, JP-A-2001-095574, and can be carried out by properly employing the methods described in these documents.

The method of the fourth invention is a method for determining an appropriate probe length for detecting a single nucleotide polymorphism of each gene using the DNA array of the first invention. Specifically, the most appropriate probe length in detecting SNP by the method of the third invention, namely the probe length capable of reflecting a single nucleotide polymorphism of each gene most exactly is the probe length determined by the fourth invention. Accordingly, the appropriate probe length can be obtained from the data provided by the method of the third invention aiming at the SNP detection of a subject.

A DNA array of the fifth invention is provided in which a probe spot having an appropriate probe length is employed in each gene. The DNA array of the fifth invention compeises "one" first probe spot and "one" second probe spot for one gene. The probes constituting each probe spot have an optimum length for detecting SNP of the gene. Accordingly, the DNA array of the fifth invention can have the probe spots for SNP detection of an enormous amount of genes in comparison to the DNA array of the first invention, while the SNP detection system is substantially the same as that of the DNA array of the first invention.

### Examples

The invention of this application is specifically illustrated in more details below by referring to Examples. However, the invention of this application is not limited by the following Examples.

### Example 1

### (1) Preparation of a DNA array

The first probes hybridizable with polynucleotides (cDNA) of genes GNB3 and MTHFR and exactly complementary to first polymorphism patterns of the respective genes, and the second probes exactly complementary to second polymorphism patterns thereof were synthesized with different lengths by a single nucleotide. The nucleotide sequences of the respective probes are as follows: for gene GNB3, the first probes (GNB3C-01 to 08) are SEQ ID NOS: 1 to 8 and the second probes (GNB3T-01 to 08) are SEQ ID NOS: 9 to 16; for gene MTHFR, the first probes (MTHFRC-01 to 08) are SEQ ID NOS: 17 to 24 and the second probes (MTHFRT-01 to 08) are SEQ ID NOS: 25 to 32. A nucleotide sequence "TTTTT" is bound to a 5' end of each nucleotide sequence of SEQ ID NOS. 1 to 32.
5' end of these probes was modified with an amino group, and 50 pmol/µL, per spot, of a solution was spotted on an oligo DNA-immobilizing epoxy glass support in amounts of 200 pL each. The first probe spots row and the second probe spots row were arranged in parallel as shown in Fig. 2 with 3 probe spots made of the same probes.
After the spotting, The DNA array was incubated overnight under conditions of 42°C and relative humidity of 50%. Subsequently, it was washed with a 0.2% SDS aqueous solution at room temperature for 2 minutes and further twice with sterile water at room temperature for 1 minute. Finally, the DNA array was incubated in sterile water of 50°C for 20 minutes, and centrifuged at 1,000 rpm for 5 minutes, followed by drying.

### (2) Preparation of labeled polynucleotides

Labeled polynucleotides were prepared by amplification under the following PCR conditions, using as a template a DNA extracted from a blood of subjects whose genotypes were identified.
· primer 1: 5 pmol (5' end labeled with Cy3)
· primer 2: 5 pmol
· x10 buffer: 2.5 µl
· 2 mM dNTP: 2.5 µl
· 25 mM MgCl₂: 2.5 µl
· Taq DNA polymerase: 1 U
· extracted DNA solution: 20 ng
· amplification conditions
   94°C/5 min
   94°C/30 sec, 60°C/30 sec, 72°C/30 sec (35 cycles)
   72°C/2 min

### (3) Hybridization

The labeled polynucleotides prepared in (2) were mixed with 0.3 N NaOH (final concentration), denatured into a single strand, and then mixed with a 200 mM citrate-phosphate buffer (pH 6.0), 2% SDS, 750 mM NaCl and 0.1% NaN₃ (which were all final concentrations) to form a sample.

Subsequently, a hybridization sample was added dropwise to the DNA array prepared in (1), and the resulting array was covered with a cover glass. The DNA array was incubated overnight in a moisture chamber of 55°C and relative humidity of 100%. After the reaction, the cover glass was removed, and the DNA array was dipped at 55° for 20 minutes in an aqueous solution of 2 x SSC and 1% SDS heated previously at 55°C. Then, the DNA array was dipped in an aqueous solution of 50 mM Tris-HCl (pH 7.5) and 0.025% Tween 20 for 15 minutes, and was centrifuged at 1,000 rpm for 5 minutes, followed by drying.

### (4) Signal measurement

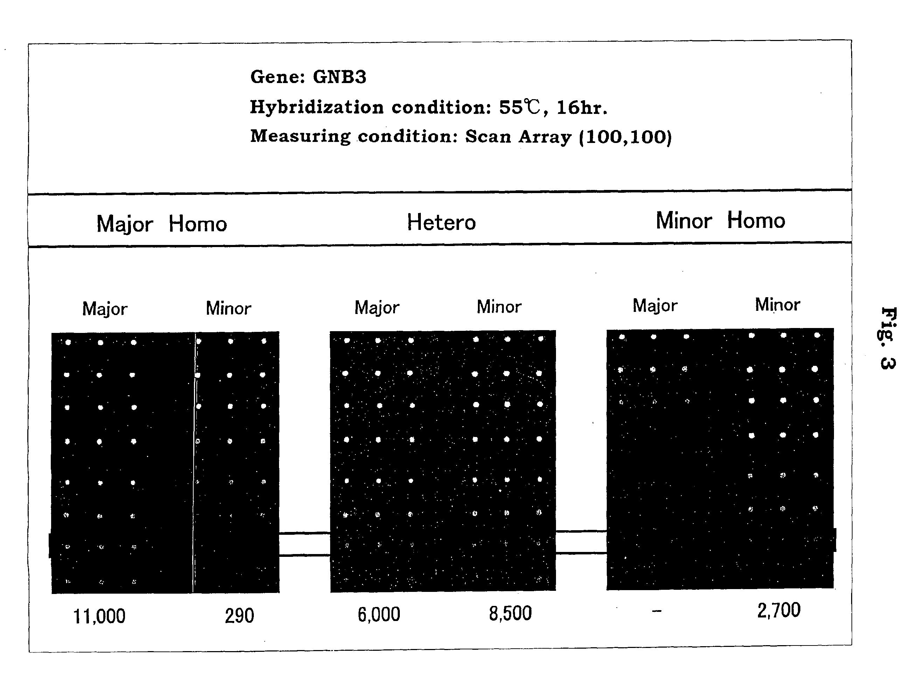

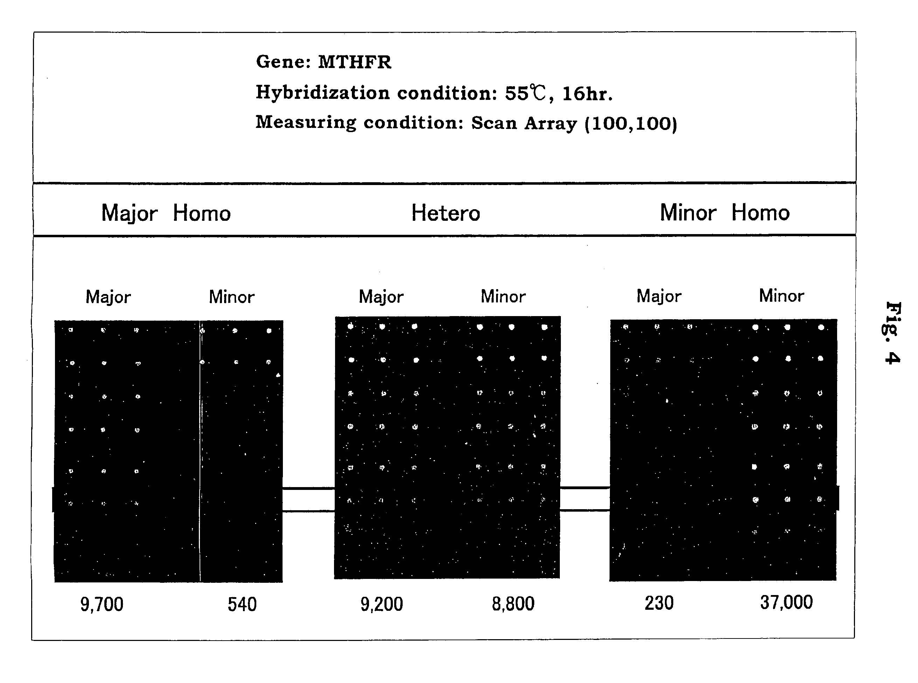

Fluorescent images were measured with Scan Array (manufactured by Packard BioScience) with a laser power of 100% and photomal of 100%. The signal images are as shown in Figs. 3 and 4. The signal images were converted to numerical values with GenePix Pro (manufactured by Axon)(Tables 1 to 3).

A cut-off value of the resulting signal is as 20,000. A fluorescent signal ratio of the first probes and the second probes (first/second ratio) was calculated, from the signals of at least one of the first probes and the second probes was 20,000 or more. When the fluorescent signal ratio was >5, the gene was judged to be a homozygotic first polymorphism pattern. When the fluorescent signal ratio was <0.2, the gene was judged to be a homozygotic second polymorphism pattern. When the fluorescent signal ratio was at least 0.2 and at most 5, the gene was judged to be a heterozygotic polymorphism pattern. Consequently, the results could be confirmed to agree with the fact.

**Table 1**

| Probe name | Signal average value | | Probe Name | Signal average value |
|---|---|---|---|---|
| GNB3C-01 | 58708 | | GNB3T-01 | 53770 |
| GNB3C-02 | 59917 | | GNB3T-02 | 61692 |
| GNB3C-03 | 51209 | | GNB3T-03 | 62803 |
| GNB3C-04 | 58654 | | GNB3T-04 | 26074 |
| GNB3C-05 | 59910 | | GNB3T-05 | 12235 |
| GNB3C-06 | 49292 | | GNB3T-06 | 4232 |
| GNB3C-07 | 11455 | | GNB3T-07 | 287 |
| GNB3C-08 | 7982 | | GNB3T-08 | - |
| | | | | |
| MTHFRC-01 | 52477 | | MTHFRT-01 | 49964 |
| MTHFRC-02 | 28936 | | MTHFRT-02 | 37235 |
| MTHFRC-03 | 24547 | | MTHFRT-03 | 682 |
| MTHFRC-04 | 23134 | | MTHFRT-04 | 402 |
| MTHFRC-05 | 19816 | | MTHFRT-05 | 1895 |
| MTHFRC-06 | 9657 | | MTHFRT-06 | 540 |
| MTHFRC-07 | 1103 | | MTHFRT-07 | 327 |
| MTHFRC-08 | - | | MTHFRT-08 | - |

**Table 2**

| Probe name | Signal average value | | Probe Name | Signal average value |
|---|---|---|---|---|
| GNB3C-01 | 43057 | | GNB3T-01 | 54237 |
| GNB3C-02 | 46372 | | GNB3T-02 | 51780 |
| GNB3C-03 | 49170 | | GNB3T-03 | 46744 |
| GNB3C-04 | 46289 | | GNB3T-04 | 48697 |
| GNB3C-05 | 39406 | | GNB3T-05 | 52358 |
| GNB3C-06 | 35536 | | GNB3T-06 | 43055 |
| GNB3C-07 | 6042 | | GNB3T-07 | 8489 |
| GNB3C-08 | 2768 | | GNB3T-08 | 216 |
| | | | | |
| MTHFRC-01 | 63347 | | MTHFRT-01 | 53418 |
| MTHFRC-02 | 54415 | | MTHFRT-02 | 59295 |
| MTHFRC-03 | 43163 | | MTHFRT-03 | 15571 |
| MTHFRC-04 | 28148 | | MTHFRT-04 | 16884 |
| MTHFRC-05 | 24618 | | MTHFRT-05 | 25909 |
| MTHFRC-06 | 9163 | | MTHFRT-06 | 8836 |
| MTHFRC-07 | 2727 | | MTHFRT-07 | - |
| MTHFRC-08 | - | | MTHFRT-08 | - |

**Table 3**

| Probe name | Signal average value | | Probe Name | Signal average value |
|---|---|---|---|---|
| GNB3C-01 | 58342 | | GNB3T-01 | 61200 |
| GNB3C-02 | 54327 | | GNB3T-02 | 60863 |
| GNB3C-03 | 8830 | | GNB3T-03 | 59395 |
| GNB3C-04 | 1245 | | GNB3T-04 | 61301 |
| GNB3C-05 | - | | GNB3T-05 | 40213 |
| GNB3C-06 | - | | GNB3T-06 | 19563 |
| GNB3C-07 | - | | GNB3T-07 | 2692 |
| GNB3C-08 | - | | GNB3T-08 | 640 |
| | | | | |
| MTHFRC-01 | 25461 | | MTHFRT-01 | 63825 |
| MTHFRC-02 | 10030 | | MTHFRT-02 | 54577 |
| MTHFRC-03 | 2625 | | MTHFRT-03 | 34217 |
| MTHFRC-04 | 1333 | | MTHFRT-04 | 46260 |
| MTHFRC-05 | 626 | | MTHFRT-05 | 54278 |
| MTHFRC-06 | 229 | | MTHFRT-06 | 37178 |
| MTHFRC-07 | - | | MTHFRT-07 | 6861 |
| MTHFRC-08 | - | | MTHFRT-08 | 979 |

### Example 2

As the same manner, polymorphisms of genes CYP 2C19-2 and CYP 2C 19-3 were tested. The nucleotide sequences of the respective probes are as follows: for gene CYP 2C19-2, the first probes (CYP 2C19-2-G-01 to 05) are SEQ ID NOS: 33 to 37 and the second probes (CYP 2C 19-2-A-06 to 010) are SEQ ID NOS: 38 to 42; for gene CYP 2C 19-3, the first probes (CYP 2C 19-3-G-O to 05) are SEQ ID NOS: 43 to 47 and the second probes (CYP 2C 19-3-A-06 to 010) are SEQ ID NOS: 48 to 52. Nucleotide sequence "TTTTT" is bound to a 5'-end of the nucleotide sequences SEQ ID NOS: 33 to 52.

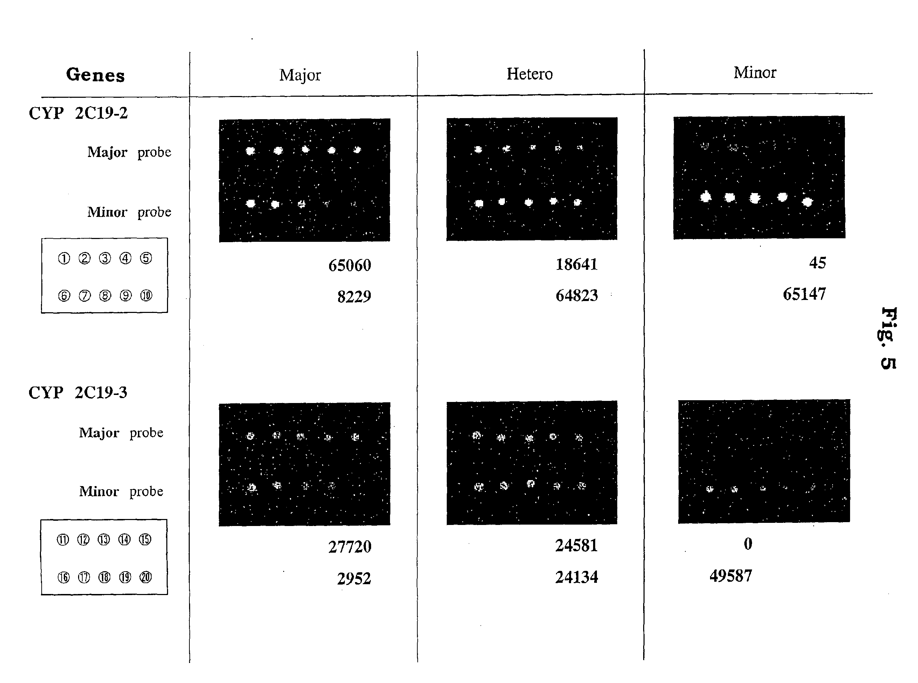

These probes were fixed on epoxy glass supports as in Example 1 (1) to form DNA microarray. The microarray constructions of genes CYP 2C 19-2 and CYP 2C 19-3 are as shown in left column of Fig. 5. That is, in the array for CYP 2C 19-2, (1) to (5) are the first probe spots row, and (6) to (10) are the second probe spots row. In the array for CYP 2C 19-3, (11) to (15) are the first probe spots row, and (16) to (20) are the second probes spot row.

Preparation of labeled polynucleotides, hybridization and signal measurement were conducted in the same manner as in Example 1 respectively.

The resulting fluorescent images are as shown in Fig. 5. The results of converting the obtained image signals into numerical values are as shown in Table 4 (CYP 2C19-2) and Table 5 (CYP 2C 19-3). A fluorescent signal ratio (first/second ratio) was calculated as in Example 1. When the fluorescent signal ratio was >5, the gene was judged to be a homozygotic first polymorphism pattern. When the fluorescent signal ratio was <0.2, the gene was judged to be a homozygotic second polymorphism pattern. When the fluorescent signal ratio was at least 0.2 and at most 5, the gene was judged to be a heterozygotic polymorphism pattern. Consequently, the results could be confirmed to agree with the fact.

**Table 4**

| Major | | | | | | |
|---|---|---|---|---|---|---|
| | Probe name | Signal value | | Probe name | Signal value | Signal ratio |
| (1) | CYP 2C 19-2-G-O 1 | 64465 | (6) | CYP 2C 19-2-A-06 | 64895 | 1.0 |
| (2) | CYP 2C 19-2-G-02 | 64984 | (7) | CYP 2C 19-2-A-07 | 65023 | 1.0 |
| (3) | CYP 2C 19-2-G-03 | 64690 | (8) | CYP 2C 19-2-A-08 | 39170 | 1.7 |
| (4) | CYP 2C 19-2-G-04 | 65309 | (9) | CYP 2C 19-2-A-09 | 13075 | 5.0 |
| (5) | CYP 2C 19-2-G-05 | 65060 | (10) | CYP 2C 19-2-A-10 | 8229 | 7.9 |

| Hetero | | | | | | |
|---|---|---|---|---|---|---|
| | Probe name | Signal value | | Probe name | Signal value | Signal ratio |
| (1) | CYP 2C19-2-G-01 | 65303 | (6) | CYP 2C 19-2-A-06 | 64989 | 1.0 |
| (2) | CYP 2C 19-2-G-02 | 64907 | (7) | CYP 2C 19-2-A-07 | 64656 | 1.0 |
| (3) | CYP 2C 19-2-G-03 | 43602 | (8) | CYP 2C 19-2-A-08 | 65083 | 0.7 |
| (4) | CYP 2C 19-2-G-04 | 48739 | (9) | CYP 2C 19-2-A-09 | 65040 | 0.7 |
| (5) | CYP 2C 19-2-G-05 | 18641 | (10) | CYP 2C 19-2-A-10 | 64823 | 0.3 |

| Minor | | | | | | |
|---|---|---|---|---|---|---|
| | Probe name | Signal value | | Probe name | Signal value | Signal ratio |
| (1) | CYP 2C 19-2-G-01 | 10371 | (6) | CYP 2C 19-2-A-06 | 64764 | 0.2 |
| (2) | CYP 2C 19-2-G-02 | 8176 | (7) | CYP 2C 19-2-A-07 | 64869 | 0.1 |
| (3) | CYP 2C 19-2-G-03 | - | (8) | CYP 2C 19-2-A-08 | 64914 | 0 |
| (4) | CYP 2C 19-2-G-04 | - | (9) | CYP 2C 19-2-A-09 | 64990 | 0 |
| (5) | CYP 2C 19-2-G-05 | - | (10) | CYP 2C 19-2-A-10 | 65147 | 0 |

**Table 5**

| Major | | | | | | |
|---|---|---|---|---|---|---|
| | Probe name | Signal value | | Probe name | Signal value | Signal ratio |
| (11) | CYP2C19-3-G-01 | 28025 | (16) | CYP 2C 19-3-A-06 | 23936 | 1.2 |
| (12) | CYP 2C 19-3-G-02 | 20753 | (17) | CYP 2C 19-3-A-07 | 25290 | 0.8 |
| (13) | CYP 2C 19-3-G-03 | 19070 | (18) | CYP 2C 19-3-A-08 | 13678 | 1.4 |
| (14) | CYP 2C 19-3-G-04 | 23035 | (19) | CYP 2C 19-3-A-09 | 8593 | 2.7 |
| (15) | CYP 2C 19-3-G-05 | 27720 | (20) | CYP 2C 19-3-A-10 | 2952 | 9.4 |

| Hetero | | | | | | |
|---|---|---|---|---|---|---|
| | Probe name | Signal value | | Probe name | Signal value | Signal ratio |
| (11) | CYP 2C 19-3-G-01 | 46011 | (16) | CYP 2C 19-3-A-06 | 44044 | 1.0 |
| (12) | CYP 2C 19-3-G-02 | 31691 | (17) | CYP 2C 19-3-A-07 | 47014 | 0.5 |
| (13) | CYP 2C 19-3-G-03 | 34519 | (18) | CYP 2C 19-3-A-08 | 28242 | 0.5 |
| (14) | CYP 2C 19-3-G-04 | 32234 | (19) | CYP 2C 19-3-A-09 | 24735 | 1.3 |
| (15) | CYP 2C 19-3-G-05 | 24581 | (20) | CYP 2C 19-3-A-10 | 24134 | 1.0 |

| Minor | | | | | | |
|---|---|---|---|---|---|---|
| | Probe name | Signal value | | Probe name | Signal value | Signal ratio |
| (11) | CYP 2C19-3-G-01 | - | (16) | CYP 2C 19-3-A-06 | 49587 | 0 |
| (12) | CYP 2C 19-3-G-02 | - | (17) | CYP 2C 19-3-A-07 | 47108 | 0 |
| (13) | CYP 2C 19-3-G-03 | - | (18) | CYP 2C 19-3-A-08 | 18297 | 0 |
| (14) | CYP 2C 19-3-G-04 | - | (19) | CYP 2C 19-3-A-09 | 9378 | 0 |
| (15) | CYP 2C 19-3-G-05 | - | (20) | CYP 2C 19-3-A-10 | 8602 | 0 |

### Industrial Applicability

As has been thus far described in detail, the invention of this application relates to a DNA array capable of more exact SNP detection and a method for conducting exact SNP detection using the DNA array. Further, the invention of this application relates to a method for determining an appropriate probe length for a gene intended for SNP detection and a DNA array having a probe with a probe length determined by this method. These inventions make it possible to search for a gene associated with susceptibity to disease or drug reactivity or to detect SNP as an important gene information for tailor-made medical care exactly with good reproducibility.

## Claims

1. A DNA array for detecting a single nucleotide polymorphism of a gene, which comprises, on a solid support,
a first probe spots group consisting of one or more probe spots each containing one or more probes hybridizable with a polynucleotide of the gene, in which the probes are exactly complementary to the first polymorphism pattern of the gene, and
a second probe spots group consisting of one or more probe spots each containing one or more probes hybridizable with the polynucleotide of the gene, in which the probes are exactly complementary to the second polymorphism pattern of the gene,
wherein probe lengths in the probe spots is different from each other.

2. The DNA array of claim 1, wherein the first probe spots group and the second probe spots group each consists of from 2 to 10 probe spots.

3. The DNA array of claim 2, wherein from 2 to 10 probe spots are arranged in the order of the probe length.

4. A kit for detecting a single nucleotide polymorphism of a gene, the kit comprising at least the DNA array of any one of claims 1 to 3.

5. A method for detecting a single nucleotide polymorphism of a gene, which uses the DNA array of any one of claims 1 to 3, and comprises the following steps of,
(a) preparing a labeled polynucleotide from the gene;
(b) contacting the labeled polynucleotide with the DNA array; and
(c) measuring signals from the labeled polynucleotide hybridized with the probes on the DNA array.

6. The method for detecting the single nucleotide polymorphism according to claim 5, wherein in the step (c) the respective signals of the first probe spots group and the second probe spots group are compared.

7. A method for determining an appropriate probe length for detecting a single nucleotide polymorphism of a gene, which uses the DNA array of any one of claims 1 to 3, comprises the following steps of,
(a) preparing a labeled polynucleotide from the gene;
(b) contacting the labeled polynucleotide with the DNA array; and
(c) measuring signals from the labeled polynucleotide hybridized with each probe on the DNA array, and
determining a probe length satisfying the following criteria as an appropriate probe length for detecting the single nucleotide polymorphism of the gene:
(i) the signal is observed in the first probe spot group in the case of the gene being homozygotic first polymorphism pattern,
(ii) the signal is observed in the second probe spot group in the case of the gene being homozygotic second polymorphism pattern, and
(iii) the signals of the same extent are observed in both of the first probe spot group and the second probe spot group in the case of the gene being heterozygotic polymorphism pattern.

8. A DNA array for detecting a single nucleotide polymorphism of a gene, which comprises, on a solid support,
a first probe spot containing one or more probes exactly complementary to the first polymorphism pattern of the gene, and
a second probe spot containing one or more probes exactly complementary to the second polymorphism pattern of the gene,
wherein the probe length is one determined by the method of claim 7.


SEQUENCE LISTING

<110> TOYOBO CO., LTD.
NGK Insulators, Ltd.

<120> DNA Array and Method for Detecting Single Nucleotide Polymorphism

<130> KAB/FP6404560

<140> EP 05721539.4
<141> 2005-03-18

<150> PCT/JP2005/005612
<151> 2005-03-18

<150> JP 2004-081034
<151> 2004-03-19

<160> 52

<210> 1
<211> 25
<212> DNA
<213> Artificial

<220>
<223> Descriptoin of artificial sequence: Synthetic oligonucleotide

<400> 1
GGCATCACGT CCGTGGCCTT CTCCC 25

<210> 2
<211> 24
<212> DNA
<213> Artificial

<220>
<223> Descriptoin of artificial sequence: Synthetic oligonucleotide

<400> 2
GCATCACGTC CGTGGCCTTC TCCC 24

<210> 3

<211>  23
<212>  DNA
<213>  Artificial

<220>
<223>  Descriptoin of artificial sequence: Synthetic oligonucleotide

<400>  3
GCATCACGTC CGTGGCCTTC TCC                                          23


<210>  4
<211>  22
<212>  DNA
<213>  Artificial

<220>
<223>  Descriptoin of artificial sequence: Synthetic oligonucleotide

<400>  4
CATCACGTCC GTGGCCTTCT CC                                           22


<210>  5
<211>  21
<212>  DNA
<213>  Artificial

<220>
<223>  Descriptoin of artificial sequence: Synthetic oligonucleotide

<400>  5
CATCACGTCC GTGGCCTTCT C                                            21


<210>  6
<211>  20
<212>  DNA
<213>  Artificial

<220>
<223>  Descriptoin of artificial sequence: Synthetic oligonucleotide

<400>  6
ATCACGTCCG TGGCCTTCTC                                              20

<210> 7
<211> 19
<212> DNA
<213> Artificial

<220>
<223> Descriptoin of artificial sequence: Synthetic oligonucleotide

<400> 7
ATCACGTCCG TGGCCTTCT 19

<210> 8
<211> 18
<212> DNA
<213> Artificial

<220>
<223> Descriptoin of artificial sequence: Synthetic oligonucleotide

<400> 8
TCACGTCCGT GGCCTTCT 18

<210> 9
<211> 25
<212> DNA
<213> Artificial

<220>
<223> Descriptoin of artificial sequence: Synthetic oligonucleotide

<400> 9
GGCATCACGT CTGTGGCCTT CTCCC 25

<210> 10
<211> 24
<212> DNA
<213> Artificial

<220>
<223> Descriptoin of artificial sequence: Synthetic oligonucleotide

<400> 10
GCATCACGTC TGTGGCCTTC TCCC 24

<210> 11
<211> 23
<212> DNA
<213> Artificial

<220>
<223> Descriptoin of artificial sequence: Synthetic oligonucleotide

<400> 11
GCATCACGTC TGTGGCCTTC TCC 23

<210> 12
<211> 22
<212> DNA
<213> Artificial

<220>
<223> Descriptoin of artificial sequence: Synthetic oligonucleotide

<400> 12
CATCACGTCT GTGGCCTTCT CC 22

<210> 13
<211> 21
<212> DNA
<213> Artificial

<220>
<223> Descriptoin of artificial sequence: Synthetic oligonucleotide

<400> 13
CATCACGTCT GTGGCCTTCT C 21

<210> 14
<211> 20
<212> DNA
<213> Artificial

<220>
<223>  Descriptoin of artificial sequence: Synthetic oligonucleotide

<400>  14
ATCACGTCTG TGGCCTTCTC                                                20


<210>  15
<211>  19
<212>  DNA
<213>  Artificial

<220>
<223>  Descriptoin of artificial sequence: Synthetic oligonucleotide

<400>  15
ATCACGTCTG TGGCCTTCT                                                 19


<210>  16
<211>  18
<212>  DNA
<213>  Artificial

<220>
<223>  Descriptoin of artificial sequence: Synthetic oligonucleotide

<400>  16
TCACGTCTGT GGCCTTCT                                                  18


<210>  17
<211>  25
<212>  DNA
<213>  Artificial

<220>
<223>  Descriptoin of artificial sequence: Synthetic oligonucleotide

<400>  17
GTCTGCGGGA GCCGATTTCA TCATC                                          25

<210> 18
<211> 24
<212> DNA
<213> Artificial

<220>
<223> Descriptoin of artificial sequence: Synthetic oligonucleotide

<400> 18
GTCTGCGGGA GCCGATTTCA TCAT 24


<210> 19
<211> 23
<212> DNA
<213> Artificial

<220>
<223> Descriptoin of artificial sequence: Synthetic oligonucleotide

<400> 19
TCTGCGGGAG CCGATTTCAT CAT 23


<210> 20
<211> 22
<212> DNA
<213> Artificial

<220>
<223> Descriptoin of artificial sequence: Synthetic oligonucleotide

<400> 20
TCTGCGGGAG CCGATTTCAT CA 22


<210> 21
<211> 21
<212> DNA
<213> Artificial

<220>
<223> Descriptoin of artificial sequence: Synthetic oligonucleotide

<400> 21

CTGCGGGAGC CGATTTCATC A                                                21

<210>  22
<211>  20
<212>  DNA
<213>  Artificial

<220>
<223>  Descriptoin of artificial sequence: Synthetic oligonucleotide

<400>  22
CTGCGGGAGC CGATTTCATC                                                  20

<210>  23
<211>  19
<212>  DNA
<213>  Artificial

<220>
<223>  Descriptoin of artificial sequence: Synthetic oligonucleotide

<400>  23
TGCGGGAGCC GATTTCATC                                                   19

<210>  24
<211>  18
<212>  DNA
<213>  Artificial

<220>
<223>  Descriptoin of artificial sequence: Synthetic oligonucleotide

<400>  24
TGCGGGAGCC GATTTCAT                                                    18

<210>  25
<211>  25
<212>  DNA
<213>  Artificial

<220>

<223>  Descriptoin of artificial sequence: Synthetic oligonucleotide

<400>  25
GTCTGCGGGA GTCGATTTCA TCATC                                          25


<210>  26
<211>  24
<212>  DNA
<213>  Artificial

<220>
<223>  Descriptoin of artificial sequence: Synthetic oligonucleotide

<400>  26
GTCTGCGGGA GTCGATTTCA TCAT                                           24


<210>  27
<211>  23
<212>  DNA
<213>  Artificial

<220>
<223>  Descriptoin of artificial sequence: Synthetic oligonucleotide

<400>  27
TCTGCGGGAG TCGATTTCAT CAT                                            23


<210>  28
<211>  22
<212>  DNA
<213>  Artificial

<220>
<223>  Descriptoin of artificial sequence: Synthetic oligonucleotide

<400>  28
TCTGCGGGAG TCGATTTCAT CA                                             22


<210>  29
<211>  21
<212>  DNA

<213>  Artificial

<220>
<223>  Descriptoin of artificial sequence: Synthetic oligonucleotide

<400>  29
CTGCGGGAGT CGATTTCATC A                                                21


<210>  30
<211>  20
<212>  DNA
<213>  Artificial

<220>
<223>  Descriptoin of artificial sequence: Synthetic oligonucleotide

<400>  30
CTGCGGGAGT CGATTTCATC                                                  20


<210>  31
<211>  19
<212>  DNA
<213>  Artificial

<220>
<223>  Descriptoin of artificial sequence: Synthetic oligonucleotide

<400>  31
TGCGGGAGTC GATTTCATC                                                   19


<210>  32
<211>  18
<212>  DNA
<213>  Artificial

<220>
<223>  Descriptoin of artificial sequence: Synthetic oligonucleotide

<400>  32
TGCGGGAGTC GATTTCAT                                                    18

<210> 33
<211> 32
<212> DNA
<213> Artificial

<220>
<223> Descriptoin of artificial sequence: Synthetic oligonucleotide

<400> 33
TTTTTTTGAT TATTTCCCGG GAACCCATAA CA 32


<210> 34
<211> 31
<212> DNA
<213> Artificial

<220>
<223> Descriptoin of artificial sequence: Synthetic oligonucleotide

<400> 34
TTTTTTGATT ATTTCCCGGG AACCCATAAC A 31


<210> 35
<211> 30
<212> DNA
<213> Artificial

<220>
<223> Descriptoin of artificial sequence: Synthetic oligonucleotide

<400> 35
TTTTTTGATT ATTTCCCGGG AACCCATAAC 30


<210> 36
<211> 29
<212> DNA
<213> Artificial

<220>
<223> Descriptoin of artificial sequence: Synthetic oligonucleotide

<400> 36

TTTTTGATTA TTTCCCGGGA ACCCATAAC 29

<210> 37
<211> 28
<212> DNA
<213> Artificial

<220>
<223> Descriptoin of artificial sequence: Synthetic oligonucleotide

<400> 37
TTTTTGATTA TTTCCCGGGA ACCCATAA 28

<210> 38
<211> 35
<212> DNA
<213> Artificial

<220>
<223> Descriptoin of artificial sequence: Synthetic oligonucleotide

<400> 38
TTTTTATTGA TTATTTCCCA GGAACCCATA ACAAA 35

<210> 39
<211> 34
<212> DNA
<213> Artificial

<220>
<223> Descriptoin of artificial sequence: Synthetic oligonucleotide

<400> 39
TTTTTATTGA TTATTTCCCA GGAACCCATA ACAA 34

<210> 40
<211> 33
<212> DNA
<213> Artificial

<220>

<223>  Descriptoin of artificial sequence: Synthetic oligonucleotide

<400>  40
TTTTTTTGAT TATTTCCCAG GAACCCATAA CAA                                33


<210>  41
<211>  32
<212>  DNA
<213>  Artificial

<220>
<223>  Descriptoin of artificial sequence: Synthetic oligonucleotide

<400>  41
TTTTTTTGAT TATTTCCCAG GAACCCATAA CA                                 32


<210>  42
<211>  31
<212>  DNA
<213>  Artificial

<220>
<223>  Descriptoin of artificial sequence: Synthetic oligonucleotide

<400>  42
TTTTTTGATT ATTTCCCAGG AACCCATAAC A                                  31


<210>  43
<211>  34
<212>  DNA
<213>  Artificial

<220>
<223>  Descriptoin of artificial sequence: Synthetic oligonucleotide

<400>  43
TTTTTATCAG GATTGTAAGC ACCCCCTGGA TCCA                               34


<210>  44
<211>  33
<212>  DNA

<213>  Artificial

<220>
<223>  Descriptoin of artificial sequence: Synthetic oligonucleotide

<400>  44
TTTTTTCAGG ATTGTAAGCA CCCCCTGGAT CCA                                33


<210>  45
<211>  32
<212>  DNA
<213>  Artificial

<220>
<223>  Descriptoin of artificial sequence: Synthetic oligonucleotide

<400>  45
TTTTTCAGGA TTGTAAGCAC CCCCTGGATC CA                                 32


<210>  46
<211>  31
<212>  DNA
<213>  Artificial

<220>
<223>  Descriptoin of artificial sequence: Synthetic oligonucleotide

<400>  46
TTTTTAGGAT TGTAAGCACC CCCTGGATCC A                                  31


<210>  47
<211>  30
<212>  DNA
<213>  Artificial

<220>
<223>  Descriptoin of artificial sequence: Synthetic oligonucleotide

<400>  47
TTTTTGGATT GTAAGCACCC CCTGGATCCA                                    30

<210>  48
<211>  35
<212>  DNA
<213>  Artificial

<220>
<223>  Descriptoin of artificial sequence: Synthetic oligonucleotide

<400>  48
TTTTTCATCA GGATTGTAAG CACCCCCTGA ATCCA                                35


<210>  49
<211>  34
<212>  DNA
<213>  Artificial

<220>
<223>  Descriptoin of artificial sequence: Synthetic oligonucleotide

<400>  49
TTTTTATCAG GATTGTAAGC ACCCCCTGAA TCCA                                 34


<210>  50
<211>  33
<212>  DNA
<213>  Artificial

<220>
<223>  Descriptoin of artificial sequence: Synthetic oligonucleotide

<400>  50
TTTTTTCAGG ATTGTAAGCA CCCCCTGAAT CCA                                  33


<210>  51
<211>  32
<212>  DNA
<213>  Artificial

<220>
<223>  Descriptoin of artificial sequence: Synthetic oligonucleotide

<400>  51

TTTTTCAGGA TTGTAAGCAC CCCCTGAATC CA 32

<210> 52
<211> 31
<212> DNA
<213> Artificial

<220>
<223> Descriptoin of artificial sequence: Synthetic oligonucleotide

<400> 52
TTTTTAGGAT TGTAAGCACC CCCTGAATCC A 31